(19) Europäisches Patentamt
European Patent Office
Office européen des brevets

(11) **EP 2 251 806 A1**

(12) **EUROPÄISCHE PATENTANMELDUNG**

(43) Veröffentlichungstag:
**17.11.2010 Patentblatt 2010/46**

(51) Int Cl.:
***G06F 19/00*** *(2006.01)*

(21) Anmeldenummer: **10157114.9**

(22) Anmeldetag: **19.03.2010**

(84) Benannte Vertragsstaaten:
**AT BE BG CH CY CZ DE DK EE ES FI FR GB GR HR HU IE IS IT LI LT LU LV MC MK MT NL NO PL PT RO SE SI SK SM TR**
Benannte Erstreckungsstaaten:
**AL BA ME RS**

(30) Priorität: **09.04.2009 DE 102009002307**
 **07.08.2009 US 232012 P**

(71) Anmelder: **Biotronik CRM Patent AG**
 **6341 Baar (CH)**

(72) Erfinder:
 • **Berdyshev, Sergey**
  **91083, Baiersdorf (DE)**

 • **Ebert, Manuel**
  **91083, Baiserdorf (DE)**
 • **Krämer, Thomas**
  **90425, Nürnberg (DE)**
 • **Meyer, Wolfgang**
  **91052, Erlangen (DE)**

(74) Vertreter: **Lindner-Vogt, Karin L.**
 **Biotronik SE & Co. KG**
 **Corporate Intellectual Properties**
 **Woermannkehre 1**
 **12359 Berlin (DE)**

(54) **Verfahren und Anordnung zur Vorhersage mindestens eines Systemereignisses sowie ein entsprechendes Computerprogramm und ein entsprechendes computerlesbares Speichermedium**

(57) Gegenstand der Erfindung ist ein Verfahren und eine Anordnung zur Vorhersage mindestens eines Systemereignisses sowie ein entsprechendes Computerprogramm und ein entsprechendes computerlesbares Speichermedium, wobei sich das Ereignis aufgrund von Trends in Observablen über einen gewissen Zeitraum vor Eintreten des Ereignisses vorhersehen lässt. Ein Ereignis kann z.B. der Ausfall eines Systems sein, weil sich das abnormale Verhalten irgendeiner Komponente in Unregelmäßigkeiten einer oder mehrerer Observablen niederschlägt oder die Früherkennung bzw. präakute Vorhersage z.B. eines bestimmten kritischen Zustandes eines Patienten.

Erfindungsgemäß ist hierfür bei Verfahren zur Vorhersage mindestens eines Systemereignisses vorgesehen, dass Messwerte $s_j$ mindestens eines Primärparameters $S_j$ innerhalb mindestens eines Zeitfensters der Länge $L$ erfasst werden, für zumindest einen Teil der Zeitfenster jeweils die Häufigkeit $\tau$ eines Trends für zumindest einen Teil des mindestens einen Primärparameters $S_j$ bestimmt wird, und auf Basis der Häufigkeit $\tau$ eines Trends eine Signalisierung eines Systemereignisses erfolgt und/oder systemereignisbezogene Maßnahmen initiiert werden.

Fig. 3a

EP 2 251 806 A1

**Beschreibung**

Gegenstand der Erfindung ist ein Verfahren und eine Anordnung zur Vorhersage mindestens eines Systemereignisses sowie ein entsprechendes Computerprogramm und ein entsprechendes computerlesbares Speichermedium, wobei sich das Ereignis aufgrund von Trends in Observablen über einen gewissen Zeitraum vor Eintreten des Ereignisses vorhersehen lässt. Das Verfahren ist bei linearen und nichtlinearen Verläufen sowie bei Sprüngen der Observablen anwendbar. Ein Ereignis kann z.B. der Ausfall eines Systems sein, weil sich das abnormale Verhalten irgendeiner Komponente in Unregelmäßigkeiten einer oder mehrerer Observablen niederschlägt oder die Früherkennung bzw. präakute Vorhersage z.B. eines bestimmten kritischen Zustandes eines Patienten. Die Systeme an sich können auch geophysikalische oder abstrakte Einheiten wie volks- oder betriebswirtschaftliche Systeme repräsentieren bei denen eine Abweichung eines definierten Normalzustandes vorhergesagt wird.

Bei komplexen Vorgängen oder Systemen werden Ereignisse durch eine Vielzahl von Faktoren ausgelöst. Diese Faktoren können ein heterogenes Auftreten aufweisen, so dass auch verschiedenartiges Verhalten von Faktoren zum selben Ereignis führen kann. Vorhersagen von Ereignissen, wie beispielsweise einer sog. Dekompensation bei Herzpatienten, sind daher bisher nur sehr ungenau möglich.

Es ist daher Aufgabe der vorliegenden Erfindung, ein Verfahren und eine Anordnung zur Vorhersage mindestens eines Systemereignisses sowie ein entsprechendes Computerprogramm und ein entsprechendes computerlesbares Speichermedium für die Vorhersage eines Ereignisses bereitzustellen, welche die genannten Nachteile vermeiden und insbesondere robust gegen Heterogenität von Signalverläufen vor bestimmten Ereignissen sind.

Diese Aufgabe wird erfindungsgemäß durch die Merkmale in den Ansprüchen 1, 9 und 13 bis 15 gelöst. Zweckmäßige Ausgestaltungen der Erfindung sind in den Unteransprüchen enthalten.

Erfindungsgemäß erfolgt die Vorhersage von Ereignissen auf Basis einiger weniger fundamentaler Annahmen, wie derjenigen, dass sich Systemveränderungen in Änderungen des Signalverlaufes niederschlagen und dass der Signalverlauf des Systems unter stabilen Verhältnissen außer Zufallsschwankungen keine systematischen Veränderungen aufweist.

Der Signalverlauf eines Systems vor einem zu detektierenden Ereignis wird in einzelne Abschnitte gegebener Länge, sog. Beobachtungsfenster unterteilt. Auf diesen Abschnitten wird eine bestimmte Form der Veränderlichkeit geprüft, indem die Hypothese getestet wird, ob in den verschiedenen Abschnitten Trends vorliegen. Die Häufigkeit der Trends für eine definierte Menge dieser Fenster wird bestimmt. Diese Größe wird für alle Messgrößen berechnet. Im Anschluss wird eine geeignete Kombination der Teilergebnisse für die Messgrößen vorgenommen und Merkmale werden konstruiert. Das Klassifikationsverfahren wird entweder mit Hilfe von Daten mit bekannten Eigenschaften optimiert oder auf unbekannte Daten angewendet und das Ergebnis des Verfahrens unter Zuhilfenahme einer maschinellen oder manuellen Entscheidungsgrenze ausgewertet.

Aus dem Ergebnis werden verschiedene Vorgehensweisen wie z. B. die Auslösung eines Alarms abgeleitet, der das Fehlverhalten eines Systems anzeigt, Abweichungen vom Normalverhalten signalisiert, automatisierte Wartungs- oder Substitutionsprozesse in Gang setzt, eine Krankheitsverschlechterung dem Arzt oder Patienten meldet, eine unterstützende Therapie, z.B. einen Schock oder die Änderung des pacing-Schemas (Stimulationsschemas) einleitet oder die Einleitung ähnlicher zeitnaher Maßnahmen erlaubt, um Schäden am System, den Ausfall des Systems oder den Tod des Patienten zu vermeiden.

Die Erfindung stellt ein Verfahren zur Vorhersage von Systemereignissen in der Zukunft bereit. Die Vorhersagen erfolgen anhand von Primärparametem, die in einem beschränktem Zeitraum vor dem Vorhersagezeitpunkt erfasst wurden, wobei aus den Primärparametem, die das System in einem bestimmten Zeitbereich charakterisieren, Merkmale gebildet werden, die die Veränderung des Systems widerspiegeln. Der Vorhersagezeitraum kann nach vom und hinten begrenzt sein und muss nicht notwendigerweise am Vorhersagezeitpunkt beginnen.

Hierfür wird innerhalb des Beobachtungszeitraums mindestens ein Zeitfenster der Länge $L$ betrachtet. Innerhalb des Zeitfensters werden Messwerte $s_j$ mindestens eines Primärparameters $S_j$ erfasst. Die Messwerte zu den Messpunkten der Primärparameter vor dem Prädiktionszeitpunkt werden in zeitlicher Reihenfolge verwendet, wobei nicht zu jedem Messpunkt der Zeitspanne gemäß Ausdruck $L^{max}+H^{max}-H^{min}$ in gültiger Messwert vorhanden sein muss. Dabei sind $L^{max}$ die größte betrachtete Fensterlänge, $H^{max}$ und $H^{min}$ der früheste bzw. späteste Vorhersagezeitpunkt.

Für zumindest einen Teil der Zeitfenster wird jeweils die Häufigkeit $\tau$ eines Trends für zumindest einen Teil des mindestens einen Primärparameters $S_j$ bestimmt. Vorzugsweise werden absteigende und/oder aufsteigende Trends betrachtet. Dabei erfolgt die Berechnung der Häufigkeiten der absteigenden und/oder aufsteigenden Trends in einer bevorzugten Ausführungsform für ein Fenster nach der Gleichung

$$\tau^{\pm} = \frac{1}{H_{max}-H_{min}} \sum_{j=1}^{i+H_{max}-H_{min}} \Theta\left(\pm C(j) - K_{j,p_0}\right),$$ wobei $\theta(x)$ die Stufenfunktion bezeichnet und $C(j) = C(L)|_{L=j}$ gemäß

$$C = \sum_{k=1}^{n-1} \sum_{l=k+1}^{n} \text{sign}(y_l - y_k) \qquad (1)$$

bestimmt wird, wobei

$$\text{sign}(y_l - y_k) = \begin{cases} 1, & y_l - y_k > 0 \\ 0, & y_l - y_k = 0 \\ -1, & y_l - y_k < 0 \end{cases}$$

die Signumsfunktion bezeichnet.

Als vorteilhaft erweist es sich, wenn zumindest ein Teil der Zeitfenster jeweils in mehrere Intervalle unterteilt wird und die Häufigkeit $\tau_i$ des Trends innerhalb der Intervalle für zumindest einen Primärparameter $S_j$ bestimmt wird. Vorzugsweise erfolgt die Unterteilung der Fenster in Intervalle in Abhängigkeit eines Primärparameters $S_j$.

Darüber hinaus erweist es sich als vorteilhaft, wenn die Häufigkeiten $\tau$ und/oder $\tau_i$ für eine Anzahl von Fenstern gemittelt werden. In einer bevorzugten Ausführungsform werden die Häufigkeiten $\tau^-$ und $\tau^+$ für jede Messgröße $S_j$ und jedes $L$ innerhalb der Intervalle $\left[ L_i^{min}, L_i^{max} \right]$ bestimmt und gemittelt. Die Mittelwerte werden mit $\hat{T}^{\pm}$ bezeichnet und berechnen sich zu $\hat{T}_i^{\pm}(S_j) = \frac{1}{n_i^L} \sum_{L=L_i^{min}}^{L_i^{max}} \tau_L^{\pm}$, $i = 1,...,N^L$, wobei $S_j$ das Messsignal Nr. $j$ und $L_i^{min}$ bzw. $L_i^{max}$ die Start- und Endpunkte der Intervalle der Länge $n_i^L$ aus der Gesamtzahl der Intervallmenge $N^L$ bezeichnen.

In einer anderen bevorzugten Ausführungsform ist vorgesehen, dass Gruppen von Primärparametern $S_j$ gebildet werden, und für mindestens eine Gruppe $q$ die Häufigkeiten $\tau$ und/oder $\tau_i$ für eine Anzahl von Fenstern gemittelt werden. Als vorteilhaft erweist es sich dabei, wenn die aus den Primärparametern gebildeten Merkmale in mehrere Gruppen $q$ analog Gleichung $\overline{T}_k^{\pm} = \frac{\frac{1}{n_i^q} \sum_{j=1}^{n_i^q} \beta_j \hat{T}_j^{\pm}(s_j)}{\sum_{j=1}^{n_i^q} \beta_j}$, $k = 1,...,N^q$ zusammengefasst werden. Dabei bezeichnet $N^G$ die Gruppenanzahl, wobei jede Gruppe $q$ eine Anzahl von $n_i^q$ Messgrößen kombiniert (mit $i = 1,..., N^q$) und eine Gruppe $q$ auch nur aus einem $\hat{T}_i^{\pm}$ bestehen kann und mit den Gewichtsfaktoren $\beta_j$ die Messgrößen nach Relevanz gewichtet gemittelt werden. Die Mittelwerte $\overline{T}_k^{\pm}$ sind sog. Merkmale und eignen sich somit als Eingangswerte für Klassifikationsverfahren $C(\overline{T}_k^{\pm})$. Die zu der gleichen Gruppe gehörenden $\overline{T}_k^+$ und $\overline{T}_k^-$ können auch als kombinierte Größe $\overline{T}_k^*$ in ein Klassifikationsverfahren einfließen. In einer bevorzugten Ausführungsform der Erfindung ist vorgesehen, dass die Merkmale individuell für ein Beobachtungsobjekt bestimmt werden. Unter Zuhilfenahme eines solchen geeigneten Klassifikationsverfahrens wird ein Indikator geliefert, anhand dessen auf das Eintreten (oder Ausbleiben) eines Ereignisses geschlossen werden kann. Ein solcher Indikator zeigt dann ein bevorstehendes Ereignis an. Bei solchen Ereignissen kann es sich beispielsweise um ein kritisches Systemereignis handeln. Bei solchen kritischen Systemereignissen kann es sich beispielsweise um Arrhythmien, Lungenembolien, Schlaganfälle, Myokardinfarkte, Angina Pectoris, Syncope, transitorische ischämische Attacken und/oder akute periphere arteriosklerotische Gefäßerkrankungen handeln.

Erfindungsgemäß ist daher vorgesehen, dass auf Basis der Häufigkeit $\tau$ eines Trends eine Signalisierung eines Systemereignisses erfolgt und/oder eine oder mehrere systemereignisbezogene Maßnahmen initiiert werden. Dies kann beispielsweise dadurch erreicht werden, dass die gemittelten Häufigkeiten $\tau$ und/oder $\tau_i$ in einem Klassifikationsverfahren ausgewertet werden und anhand der Klassifikation die Vorhersage des mindestens einen Systemereignisses erfolgt. Wie erwähnt, kann vorteilhafterweise beispielsweise der auf Basis der Häufigkeit $\tau$ eines Trends berechnete Mittelwerte $\overline{T}_k^{\pm}$ in ein Klassifikationsverfahren einfließen. Bei solchen Maßnahmen kann es sich beispielsweise um systemstabilisierende Maßnahmen handeln, wobei systemstabilisierende Maßnahmen Medikamentierungsänderungen und/oder andere therapeutische Unterstützungsmaßnahmen, automatisierte Wartungs- und/oder Substitutionsprozesse umfassen.

Eine bevorzugte Ausführungsform sieht vor, dass die Klassifikationsverfahren unter Verwendung von klassifizierten Ereignisdaten und/oder von Kontrolldaten optimiert werden. Als vorteilhaft erweist es sich auch, wenn scharfe oder unscharfe Klassifikationsgrenzen verwendet werden.

Eine andere bevorzugte Ausführungsform sieht vor, dass die Bestimmung des Trends die Prüfung einer Hypothese auf das Vorliegen des Trends umfasst. Vorzugsweise wird dabei die oben erwähnte Größe C mit dem $K_{n,p}$ p-Quantil $k_{n,p}$ der Kendall'schen K-Statistik verglichen, um das Auftreten eines negativen bzw. positiven Trends mit der Wahrscheinlichkeit $p \geq p_s$ zu ermitteln.

Vorzugsweise werden die Messwerte (= Messsignale) $s_j$ des mindestens einen Primärparameters $S_j$ in bestimmten Zeitintervallen erfasst. Die Einheiten der Zeitintervalle können Sekunden, Minuten, Stunden, Tage, Wochen, Monate oder Jahre sein, wobei die Zeitintervalle für die verschiedenen Primärparameter verschieden sein können.

Mit der Erfindung können beispielsweise heterogene Signalverläufe ausgewertet werden. Die Signalverläufe können dabei linear, nichtlinear oder nicht stetig sein.

Ein weiterer Vorteil der Erfindung besteht darin, dass das Verfahren robust gegenüber Datenlücken ist.

Als Primärparameter kommen beispielsweise aber nicht ausschließlich folgende in Frage:

Alle Primärparameter, die die Herzrate des Patienten beinhalten, wie z.B. die Herzrate über einen vordefinierten Zeitraum, die Herzrate während einer definierten Ruhephase, die Variabilität der Herzrate und ähnliche. Alle Primärparameter, die Impedanzen im Patienten bestimmen, sei es durch intrakardiale (bipolare und multipolare), intrathorakale Messungen oder durch Bestimmung mit externen Sensoren. Alle Primärparameter, die die Aktivität eines Patienten auf irgendeine Weise bestimmen. Alle Primärparameter, in denen der Anteil links- oder rechtsventrikulärer stimulierter, wahrgenommener oder anderer Events einfließt. Alle Primärparameter, die implantatabhängige Signale aufzeichnen. Alle Primärparameter, die Extrasystolen unabhängig ihres Ursprungorts bestimmen. Alle Primärparameter, die die Hämodynamik eines Patienten oder sonstige Elastizitäten, Drücke, Volumina oder Abstände erfassen. Alle Primärparameter, die außerhalb eines Implantats bestimmt werden, wie Größen, die durch aus Funk angebundenen Sensoren gewonnen werden oder Größen von Geräten, die Daten außerhalb des Körpers aufzeichnen und diese telemetrisch der Auswertungseinheit übermitteln. Alle biomedizinischen Primärparameter wie Stimulationsschwellen, Elektrodenkonfigurationen, Sensorverstärkungen oder Versatzwerte. Alle Primärparameter wie Blutzuckerspiegel, andere Biomarker oder ähnliche Größen. Alle Primärparameter, die biometrische Informationen über den Patienten bestimmen. Alle Primärparameter, die zusätzliche Informationen über eine Medikamentierung aufzeichnen. Alle Primärparameter die aus elektrophysiologischen oder biochemischen Verfahren bestimmt werden. Alle Primärparameter, die Signale aus bildgebenden, akustischen oder mechanischen Verfahren erfassen. Alle oben genannten Primärparameter, die zuvor normiert und/oder skaliert wurden. Alle möglichen Kombinationen mehrerer der oben genannten Primärparameter.

Eine Anordnung nach der Erfindung weist mindestens einen Chip und/oder Prozessor auf und ist derart eingerichtet, dass ein Verfahren zur Vorhersage mindestens eines Systemereignisses ausführbar ist, wobei Messwerte $s_j$ mindestens eines Primärparameters $S_j$ innerhalb mindestens eines Zeitfensters der Länge L erfasst werden, für zumindest einen Teil der Zeitfenster jeweils die Häufigkeit τ eines Trends für zumindest einen Teil des mindestens einen Primärparameters $S_j$ bestimmt wird, und auf Basis der Häufigkeit τ eines Trends eine Signalisierung eines Systemereignisses erfolgt und/oder systemereignisbezogene Maßnahmen initiiert werden.

Ein Computerprogramm zum Vorhersagen von Systemereignissen ermöglicht es einer Datenverarbeitungseinrichtung, nachdem es in den Speicher der Datenverarbeitungseinrichtung geladen worden ist, ein Verfahren zur Vorhersage mindestens eines Systemereignisses durchzuführen, wobei Messwerte $s_j$ mindestens eines Primärparameters $S_j$ innerhalb mindestens eines Zeitfensters der Länge L erfasst werden, für zumindest einen Teil der Zeitfenster jeweils die Häufigkeit τ eines Trends für zumindest einen Teil des mindestens einen Primärparameters $S_j$ bestimmt wird, und auf Basis der Häufigkeit τ eines Trends eine Signalisierung eines Systemereignisses erfolgt und/oder systemereignisbezogene Maßnahmen initiiert werden.

In einer weiteren bevorzugten Ausführungsform der Erfindung ist vorgesehen, dass das erfindungsgemäße Computerprogramm modular aufgebaut ist, wobei einzelne Module auf verschiedenen Datenverarbeitungseinrichtungen installiert sind.

Vorteilhafte Ausführungsformen sehen zusätzlich Computerprogramme vor, durch welche weitere in der Beschreibung angegebene Verfahrensschritte oder Verfahrensabläufe ausgeführt werden können.

Solche Computerprogramme können beispielsweise (gegen Gebühr oder unentgeltlich, frei zugänglich oder passwortgeschützt) downloadbar in einem Daten- oder Kommunikationsnetz bereitgestellt werden. Die so bereitgestellten Computerprogramme können dann durch ein Verfahren nutzbar gemacht werden, bei dem ein Computerprogramm nach Anspruch 13 aus einem elektronischen Datennetz, wie beispielsweise aus dem Internet, auf eine an das Datennetz angeschlossene Datenverarbeitungseinrichtung heruntergeladen wird.

Um das erfindungsgemäße Verfahren zur Vorhersagen von Systemereignissen durchzuführen, ist vorgesehen, ein computerlesbares Speichermedium einzusetzen, auf dem ein Programm gespeichert ist, das es einer Datenverarbeitungseinrichtung ermöglicht, nachdem es in den Speicher der Datenverarbeitungseinrichtung geladen worden ist, ein Verfahren zur Vorhersage mindestens eines Systemereignisses durchzuführen, wobei Messwerte $s_j$ mindestens eines Primärparameters $S_j$ innerhalb mindestens eines Zeitfensters der Länge $L$ erfasst werden, für zumindest einen Teil der Zeitfenster jeweils die Häufigkeit $\tau$ eines Trends für zumindest einen Teil des mindestens einen Primärparameters $S_j$ bestimmt wird, und auf Basis der Häufigkeit $\tau$ eines Trends eine Signalisierung eines Systemereignisses erfolgt und/ oder systemereignisbezogene Maßnahmen initiiert werden.

Nachfolgend sollen nähere Einzelheiten von vorteilhaften Ausführungsformen der Erfindung erläutert werden:

Grundlegende Berechnung (Merkmalskonstruktion):

Im ersten Schritt werden die einlaufenden Messsignale $s_j$ der Messgröße $S_j$ einer Filterung unterzogen. Die Messgröße $S_j$ mit $j = 1,...,N^S$ geht im Folgenden als sog. Primärparameter in die Merkmalskonstruktion ein. Die Anzahl der Primärparameter wird mit $N^S$ bezeichnet (hochgestellte Zeichen und Ziffern sind Indices, wenn nicht anders vermerkt). Im ersten Schritt wird u.a. geprüft, ob die Werte der Messgrößen in einem gültigen Wertebereich liegen.

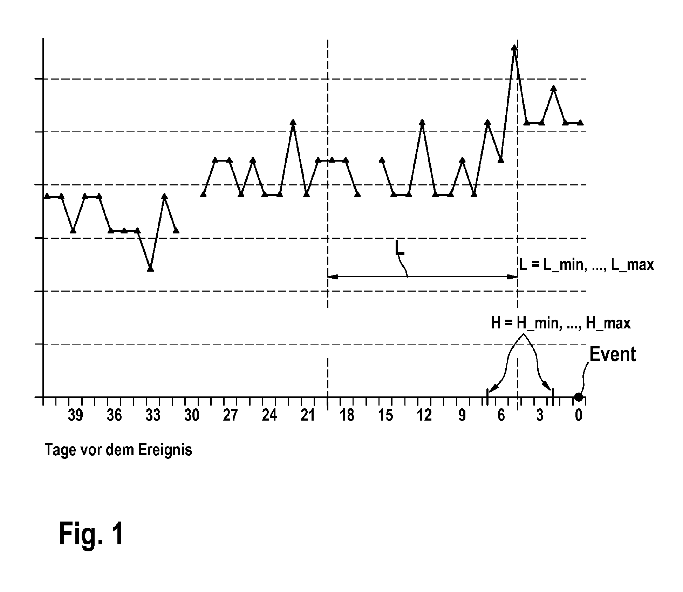

Die Zeitreihe der Messsignale ist von zwei Parametern abhängig, deren Bedeutung in Figur 1 skizziert ist. Die Variable $L \in \mathbb{N}$ stellt dabei ein Beobachtungsfenster zwischen $L_{min} \in \mathbb{N}$ und $L_{max}, \in \mathbb{N}$ dar und in dessen Verlauf wird die Veränderlichkeit eines Messsignals geprüft. Die Variable $H \in \mathbb{N}$ beschreibt die Vorlaufzeit zwischen dem Zeitpunkt der Prädiktion und dem Eintreten des vorhergesagten Ereignisses, also z.B. die Zeit bis zum tatsächlichen oder vermuteten Auftreten des bevorstehenden Dekompensationsprozesses und läuft von $H_{min} \in \mathbb{N}$ bis $H_{max} \in \mathbb{N}$. Das Fenster sollte anschließend mit einem geeigneten Kriterium auf Gültigkeit überprüft werden. Eine Möglichkeit wäre die Forderung nach mind. 50% validen Messsignalwerten.

Für gültige Fenster wird der Test auf Trend nach Mann verwendet. Hierbei wird für jedes Fenster die Größe

$$C = \sum_{k=1}^{n-1} \sum_{l=k+1}^{n} \mathrm{sign}(y_l - y_k) \quad (1)$$

berechnet, wobei

$$\mathrm{sign}(y_l - y_k) = \begin{cases} 1, & y_l - y_k > 0 \\ 0, & y_l - y_k = 0 \\ -1, & y_l - y_k < 0 \end{cases}$$

die Signumsfunktion bezeichnet.

Die Hypothese eines negativen Trends wird bestätigt, falls

$$C < -K_{np},$$

die Hypothese eines positiven Trends, falls

$$C > K_{np}.$$

$K_{np}$ ist hierbei das $p$-Quantil der Kendall'schen $K$-Statistik. Das Trendergebnis wird für jedes Parametertupel $(L, H)$ einzeln berechnet.

Die Variable $L$ kann zudem in eine Menge von $N^1$ nicht notwendigerweise disjunkten Intervallen der Länge

$n_i^L$ mit $i = 1,..., N^L$ unterteilt werden. Hierbei sind $L_i^{min} \in \mathbb{N}$ und $L_i^{max} \in \mathbb{N}$ die Start-, bzw. Endpunkte dieser Intervalle der Länge $n_i^L$. Dabei gehört zu jedem $S_j$ ein $N^L$ und ein $n_i^L$ (ein zusätzlicher Index $j$ wird zur Vereinfachung nur dort erwähnt wo er zwingend erforderlich ist).

Anschließend werden die Häufigkeiten $\tau^-$ und $\tau^+$ des Auftretens eines negativen bzw. positiven Trends mit der Wahrscheinlichkeit $p \geq p_s$ für ein Fenster $L$ über alle $H$ zwischen $H_{min}$ und $H_{max}$ gemittelt, also von $L$ bis $L+H_{max}-H_{min}$. $p_s$ muss entsprechend der gewünschten Zuverlässigkeit des Gesamtverfahrens vorgegeben werden. $\tau^{\pm}$ berechnet sich also zu

$$\tau^{\pm} = \frac{1}{H_{max} - H_{min}} \sum_{j=L}^{L+H_{max}-H_{min}} \theta\left(\pm C(j) - K_{j,p_s}\right), \quad (2)$$

wobei

$$\theta(x) = \begin{cases} 0, x < 0 \\ 1, x \geq 0 \end{cases}$$

die Theta-Funktion bezeichnet.

Die Häufigkeiten $\tau^-$ und $\tau^+$ werden für jede Messgröße $S_j$ und jedes $L$ innerhalb der Intervalle $\left[L_i^{min}, L_i^{max}\right]$ bestimmt und gemittelt. Die Mittelwerte werden mit $\tilde{T}^{\pm}$ bezeichnet und berechnen sich zu

$$\tilde{T}_i^{\pm}(S_j) = \frac{1}{n_i^L} \sum_{L=L_i^{min}}^{L_i^{max}} \tau_L^{\pm} \quad, i = 1,...,N^L, \quad (3)$$

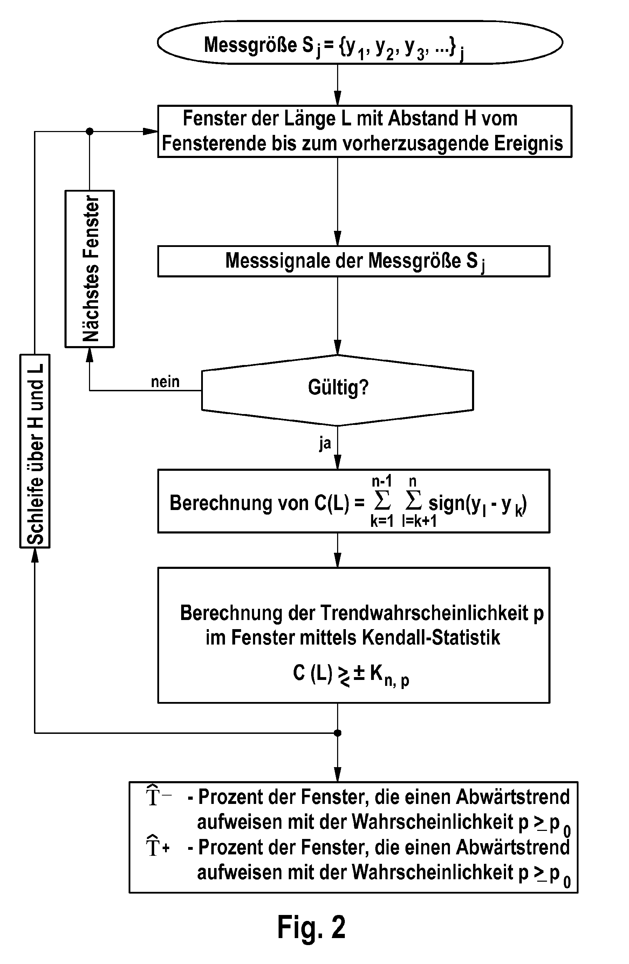

Figur 2 zeigt schematisch das Zustandekommen dieser Mittelwerte.

In einem weiteren Schritt werden mehrere Messgrößen zu einer Gruppe $q$ zusammengefasst, so dass $N^q$ Gruppen entstehen, wobei jede Gruppe $q$ $n_i^q$ Messgrößen kombiniert (mit $i = 1,..,N^q$) und wobei eine Gruppe $q$ auch nur aus einem $S_j$ bestehen kann. Die Häufigkeiten $\tilde{T}_i^{\pm}$ werden für jede Messgröße innerhalb einer Gruppe $q$ bestimmt, mit den Gewichtsfaktoren $\beta_j$ nach Relevanz gewichtet gemittelt. Diese Mittelwerte werden mit $T_k^{\pm}$ bezeichnet und berechnen sich zu

$$T_k^{\pm} = \frac{\dfrac{1}{n_k^q} \sum_{j=1}^{n_k^q} \beta_j \hat{T}_j^{\pm}(S_j)}{\sum_{j=1}^{n_k^q} \beta_j}, \, k = 1,...,N^q. \quad (4)$$

Es wird gefordert, dass $L_i^{min}(S_i) = L_j^{min}(S_j)$, bzw. $L_i^{max}(S_i) = L_j^{max}(S_j)$ für alle in der Summe berücksichtigten Messsignale gilt. In Gleichung (4) wird das arithmetische Mittel zur Mittelung als Lageparameter verwendet. An dieser Stelle kann auch ein anderer Lageparameter, wie z.B. der Median sinnvoll eingesetzt werden.

Es ergibt sich eine diagnostische Merkmalsmenge der Größe

$$2 \sum_{j=1}^{N^q} N_j^L \, ,$$

Die Summation folgt aus der Tatsache, dass $N^L$ für jede Gruppe $q$ verschieden sein kann.

Die Mittelwerte $\overline{T_k^{\pm}}$ sind sog. Merkmale und eignen sich somit als Eingangswerte für Klassifikationsverfahren $\mathcal{C}(\overline{T_k^{\pm}})$. Die zu der gleichen Gruppe gehörenden $\overline{T_k^+}$ und $\overline{T_k^-}$ können auch als kombinierte Größe $\overline{T_k^{\circ}}$ in ein Klassifikationsverfahren einfließen.

Optimierungsprozess:

Um die Merkmalsmenge dem Auftreten von Ereignissen zuordnen zu können, ist es erforderlich, ein Klassifikationsverfahren $\mathcal{C}$ einzusetzen, das selbst wiederum angepasst werden muss. Für diese Optimierung sind für jeden eingesetzten Primärparameter klassifizierte Ereignisdaten über einen Zeitraum von $L^{max} + H^{max}$ unmittelbar vor dem gesicherten Ereignis und z.B. entsprechende Kontrolldatensätze der gleichen Länge ohne Ereignis erforderlich. Für eine möglichst hohe Güte des Seperationsverfahrens ist die Verwendung von Datensätzen mit gesicherten Ereignissen und unter Umständen eine sorgfältige Auswahl der Kontrolldaten erforderlich.
Es wird nun für jede der Parametergruppen $q$ das Merkmal $T^{\pm}$ bestimmt. Derartig klassifizierte Merkmale eignen sich für automatisiertes Lernen in Abhängigkeit der Anzahl von Ereignis- und Kontrolldatensätzen. Hier haben sich Verfahren wie künstliche neuronale Netze, Support Vector Machines oder ähnliche bewährt. Aufgrund der hohen Anzahl von Sätzen freier Parameter während des Trainingsprozesses wird bei diesen Verfahren eine große Anzahl klassifizierter Merkmale benötigt.
Liegen diese Merkmale noch nicht in der für diese Verfahren benötigten Häufigkeit vor, so ist ein Klassifikationsverfahren zu verwenden, bei dem die Anzahl freier Parameter mit der Anzahl zur Verfügung stehender Trainingsdatensätzen verträglich ist. Im Folgenden soll exemplarisch eine solche Möglichkeit vorgestellt werden:

Zunächst wird aus den erhaltenen Merkmalen ein Merkmalsvektor

$$\vec{T} = \left( \overline{T_{1,1}^-} , \overline{T_{1,1}^+} , ... , \overline{T_{N^q,N^L}^-} , \ \overline{T_{N^q,N^L}^+} \right)$$

gebildet, der dann skalar mit einem Klassifikationsvektor

$$\vec{a} = \left( \alpha_{1,1}^- , \alpha_{1,1}^+ , ... , \alpha_{N^q,N^L}^- , \ \alpha_{N^q,N^L}^+ \right)$$

multipliziert wird. Das Ergebnis dieser skalaren Multiplikation ist eine Maßzahl für das Auftreten eines Ereignisses. Während des Trainingsprozesses wird für Event- bzw. Kontrolldaten der Ausgangswert auf einen fixen Wert $c_{Event}^{Train}$ bzw. $c_{Control}^{Train}$ gesetzt, der überhalb, bzw. unterhalb des Trainingsschwellwertes $c_{o,Train}$ gelegt wird. Im anschließenden Testverfahren muss ein $c_{o,Class}$ gefunden werden, damit das Verfahren eine vorgegebene Spezifität und/ oder Sensitivität erreicht, zusammen mit der weiter oben gewählten Trendwahrscheinlichkeit $p_0$. Für die Komponenten des Klassifikationsvektors $\vec{a}$ können diskrete Werte (z.B, -1, 0, +1 oder ein feineres Gitter) vorgegeben werden, um die Anzahl der Freiheitsgrade weiter einzuschränken. Es kann auch durch systematische Variation der Komponenten von $\vec{a}$ ein maximaler Abstand zwischen $c_{event}$ und $c_{control}$ erreicht werden. Die in diesem Beispiel eingesetzte Klassifikationsfunktion hat die Form

$$c_{Bsp} = \sum_{j=1}^{N^L} \left[ \sum_{k=1}^{N^S} \left( \alpha_{k,j}^- T_{k,j}^- + \alpha_{k,j}^+ T_{k,j}^+ \right) \right]$$

Im Allgemeinen besteht die Einschränkung in diesem Beispiel auf einen skalaren Schwellenwert nicht, so dass als Entscheidungsgrenze eine Hyperebene im $\mathbb{R}^S$ zu verwenden ist.

Anwendung des Verfahrens:

Nach der Optimierung kann das Klassifikationsverfahren zur Vorhersage eines Ereignisses für die Zeitspanne $H^{min}$ bis $H^{max}$ vom Prädiktionszeitpunkt aus gesehen in der Zukunft liegend eingesetzt werden. Einzige Vorraussetzung sind

$$L^{max} + H^{max} - H^{min} \quad (5)$$

Messwerte. Die daraus erhaltenen Merkmale $T_k^\pm$ können als Input für ein gewähltes Klassifikationsverfahren $C$ dienen. Ein System kann nun über die Zeit überwacht werden, das Verfahren wird nach jedem Eintreffen eines neuen Messwertes oder in größeren Abständen erneut in Gang gesetzt, um eine Vorhersage über Normabweichungen des Systems zu treffen. Wird der im Rahmen des Optimierungsverfahrens bestimmte Schwellenwert $c_{o,Class}$ über- oder unterschritten, so lassen sich vordefinierte Maßnahmen, wie z.B. eine therapeutische Aktionskette in Gang setzen.

Umgang mit ungültigen und/oder fehlenden Messwerten:

Vor dem Erzeugen der Merkmale werden die zur Merkmalsberechnung herangezogenen Messwerte der verwendeten Messgrößen einer Gültigkeitsprüfung unterzogen. Einzelne Messwerte können entweder fehlen, da sie z.B. innerhalb der Übertragungsstrecke auf irgendeine Weise verloren gehen oder sie können nach einer Validitätsprüfung als ungültig markiert werden, da sie z.B. außerhalb eines vordefinierten Wertebereiches liegen. Das Verfahren kann ungültige und fehlende Messwerte z.B. auf folgende Weise handhaben:

- Beim Test auf Trend nach Mann nach Gleichung (1) werden derartige Messwerte ignoriert, indem sich in diesem Fall die effektive Fensterlänge um die Anzahl dieser Messwerte reduziert. Die Verkürzung der Fensterlänge muss in diesem Fall auch in Gleichung (2) berücksichtigt werden.
- Ungültige und/oder invalide Messwerte werden durch entsprechende Werte aus einem Interpolationsverfahren ersetzt.
- Eine andere Möglichkeit zur Handhabung von missing values wird verwendet.

Nachstehend wird die Erfindung an einem Ausführungsbeispiel anhand der beigefügten Zeichnungen näher erläutert. Es zeigen:

Figur 1: Beschreibung der Parameter $L_{min}$, $L_{max}$, $H_{min}$ und $H_{max}$;

Figur 2: Schema vom Einlaufen der Messdaten bis zur Bestimmung der summarischen Trendhäufigkeiten;

Figur 3a: ein erstes Beispiel eines Prädiktorverlaufes über die Zeit und

Figur 3b: ein zweites Beispiel eines Prädiktorverlaufes über die Zeit.

Als Ausführungsbeispiel dient ein Gerät zur Vorhersage einer sog. Dekompensation bei Patienten, die an chronischem Herzversagen bzw. Stauungsinsuffizienz des Herzens (CHF, chronic/congestive heart failure) leiden. Die Erfindung ist jedoch nicht auf den medizinischen Bereich eingeschränkt. Vielmehr ist sie auch nutzbar, um Ereignisse in andere komplexen Systemen, wie etwa meteorologischen, geophysikalischen Systemen oder auch in volks- oder betriebswirtschaftlichen Systemen.

Kennzeichnend für diese Patienten ist, dass trotz eines generell chronischen Verlaufs der Erkrankung zeitweise Phasen eines akuten und kurzfristig lebensbedrohlichen Zustandes eintreten, die eine Hospitalisierung erfordern. Da die Hospitalisierung mit hohen Kosten und das Eintreten einer Dekompensation mit höherer Mortalität und Morbidität verbunden ist, bemüht man sich um deren Vermeidung durch einen frühzeitigen therapeutischen Eingriff. Voraussetzung dafür ist das frühzeitige Erkennen einer möglichen Dekompensation.

Eine Reihe von CHF-Patienten bietet Indikationen zur Implantation eines elektrisch aktiven Implantates. Dies ist meist ein CRT-Schrittmacher (CRT = Cardiac Resynchronization Therapy), um durch eine Wiederherstellung der Synchronisation der Herzkammern die Ursachen einer Pumpschwäche des Herzens direkt zu bekämpfen. Zur Absicherung gegen den plötzlichen Herztod kann bei myokardialer Insuffizienz auch ein automatischer Defibrillator implantiert werden. Bei entsprechenden technischen Einrichtungen ist es möglich, über die Sonden und Sensoren dieser Implantate verschiedene Messgrößen physiologischer oder technischer Natur zu bestimmen. Diese Messgrößen lassen sich entweder direkt in dem Implantat verarbeiten oder z.B. in einem externen Rechensystem auswerten, indem die Messgrößen vom Implantat über ein Patientengerät an den behandelnden Arzt übertragen werden. Dabei kommen auch andere Implantate oder externe Gerätetypen in Frage.

Da somit ein Anteil von CHF-Patienten derzeit ein elektrisch aktives Implantat (CRT-Schrittmacher, ICD = Implantable Cardioverter Defibrillator) erhält, bietet es sich an, die Früherkennung einer CHF-Dekompensation im Rahmen einer Homemonitoring-Lösung anzustreben. Die hierzu ausgeführte Methodik ist nicht zwingend an Implantate gebunden.

Die Sensoren umfassen je nach Ausführung Elektroden zur Erfassung des intrakardialen Elektrogrammes, zur Bestimmung von Impedanzen, des Druckes und weitere Messgrößen. Die Messgrößen ihrerseits lassen sich einer optionalen Zwischenverarbeitung unterziehen oder direkt einer Analyse im Hinblick auf die Diagnose eines Patienten zuführen. Die Erzielung von belastbaren diagnostischen Aussagen ist jedoch mit einer Reihe von Schwierigkeiten behaftet.

Die Grunderkrankung CHF beschreibt einen komplexen pathophysiologischen Prozess, dem vielfältige Ursachen zu Grunde liegen. Unterschieden werden dabei z. B. ischämische, nicht-ischämische, dilatative, toxische oder idiopathische Auslöser von CHF. Faktoren, wie z. B. mangelhafte Patientencompliance bei Medikamenteneinnahme oder Einhaltung der Diät, akute Atemwegserkrankungen, Infektionen oder andere Komorbiditäten erschweren zusätzlich die Beschreibung dieser Grunderkrankung.

Wegen der Komplexität der Grunderkrankung und dem Dekompensationsverlauf sind die gemessenen Patientensignale heterogen vor Auftreten der zu beobachtenden Ereignisse. Dies bedeutet, dass bei verschiedenen Patienten auch ein unterschiedlicher Signalverlauf zu erwarten ist, und ebenso wird auch der Verlauf zweier verschiedener Dekompensationsereignisse bei ein und demselben Patienten unterschiedlich ausfallen. In beiden Fällen können zudem Lücken in der Messreihe auftreten. Ursache hierfür können Verluste auf der Telemetriestrecke oder Verwerfen von Messwerten nach einer Gültigkeitsprüfung sein.

Die Heterogenität im Signalverlauf vor Ereignissen erstreckt sich auf mehrere verschiedene Aspekte:

- Erstens treten Unterschiede dahin gehend auf, in welcher Messgröße sich Änderungen widerspiegeln. So kann es sein, dass erste Ansätze für eine Dekompensation sich zuerst im Niveau der mittleren Herzrate zeigen, Impedanzänderungen erst im Rahmen der eigentlichen Dekompensation auftreten, es kann jedoch auch umgekehrt sein.
- Zweitens ist die Reaktion des Patienten auf eine beginnende Dekompensation in gewissem Maße verhaltensabhängig. Dies bedeutet, dass einzelne Messgrößen bei einer Gruppe von Patienten ansteigen, während sie bei einer anderen Gruppe abfallen, wenn die eine z.B. aufgrund von Nervosität aktiver wird, die andere aus Ängstlichkeit sich eher zurückgezogen verhält.
- Drittens wird sich die Progression des Krankheitsverlaufes vor einer Dekompensation im Allgemeinen hinsichtlich des Zeitverlaufes unterscheiden, der von der individuellen Konstitution, dem Verhalten oder anderen Faktoren abhängt. Insbesondere können weitere nicht bekannte, nicht messbare, nicht kontrollierbare und individuelle Faktoren in der Grundgesamtheit der CHF-Patienten auftreten.

Es ist erforderlich eine kontinuierliche Überwachung zu gewährleisten, d. h. nach Möglichkeit mindestens einmal am Tag Messungen durchzuführen. Zudem sind Belastungen für den Patienten und Kosten zu vermeiden, die entstehen können, wenn zu häufig Fehlalarme auftreten und daraus folgend der Patient zu häufig kontaktiert und/oder hospitalisiert wird oder sogar überflüssige diagnostische und/oder inadäquate therapeutische Eingriffe vorgenommen werden. Die Verfahren müssen daher auch eine hohe Spezifität, d. h. eine sehr niedrige Fehlalarmquote aufweisen und daher eine zuverlässige unterstützende Entscheidungsgrundlage für den Arzt bereitstellen. Zusätzlich soll mit einer hohen Sensitivität die Dekompensation eines möglichst breiten Patientenkollektivs erkannt werden.

Zur Gewährleistung einer hinreichenden statistischen Güte ist es daher erforderlich, ein Verfahren zu verwenden, das robust gegenüber den oben genannten Heterogenitäten ist. Dabei kommt aufgrund der Natur der Erkrankung hinzu, dass ein konkretes Verlaufsmuster einer Dekompensation für den jeweiligen Patienten nicht a-priori bekannt sein kann.

Als Annahmen lassen sich treffen, dass

- sich pathophysiologische Änderungen des kardiovaskulären Substrates in Signaländerungen im Vergleich zum Normalzustand niederschlagen müssen
- dass unter stabilen physiologischen Verhältnissen auch die beobachtbaren Messgrößen außer Zufallsschwankungen keine systematischen Veränderungen aufweisen dürfen.

Diese beiden Annahmen stellen den wesentlichen Grundsatz dar, auf dem die in diesem Abschnitt beschriebene Variante eines geeigneten Algorithmus beruht.

Im Folgenden soll durch eine exemplarische Wahl der Parameterwerte die Handhabung des Verfahrens veranschaulicht werden.

Beobachtungsfensters: $L_{min}$ = 8, $L_{max}$ = 28

Vorlaufzeit der Prädiktion: $H_{min}$ = 2, $H_{max}$ = 12

Anzahl Primärparameter: $N^S$ = 8

geforderte Trendwahrscheinlichkeit: $p_0$ = 0.99

Anzahl der Intervalle: $N^L = N^L_j = 3$ für jeden Primärparameter $S_j$

Start- und Endpunkt der Intervalle:

$$L_1^{min} = 8 \; , \quad L_1^{max} = 14 \; , \; L_2^{min} = 15 \; , \; L_2^{max} = 21 \; , \; L_3^{min} = 22 \; , \; L_3^{max} = 28$$

Länge der Intervalle: $n^L_{1,j} = n^L_{2,j} = n^L_{3,j} = 7$ für jeden Primärparameter $S_j$

Anzahl der Primärparametergruppen: $N^q$ = 4

Anzahl Primärparameter pro Gruppe: $n_1^q = 3, n_2^q = 1, n_3^q = 3, n_4^q = 1$

Gewichtungsfaktoren: $\beta_1 = \beta_2 = \beta_3 = \beta_4 = 1$

Größe der diagnostischen Merkmalsmenge: $2 \sum_{j=1}^{N^S} N_j^L = 24$

Die Primärparameter können aus folgender Menge gewählt werden:

- Alle Messgrößen, die die Herzrate des Patienten beinhalten, wie z.B. die Herzrate über einen vordefinierten Zeitraum, die Herzrate während einer definierten Ruhephase, die Variabilität der Herzrate und ähnliche
- Alle Größen, die Impedanzen im Patienten bestimmen, sei es durch intrakardiale (bipolare und multipolare), intrathorakale Messungen oder durch Bestimmung mit externen Sensoren
- Alle Messgrößen, die die Aktivität eines Patienten auf irgendeine Weise bestimmen
- Alle Messgrößen, in denen der Anteil links- oder rechtsventrikulärer stimulierter, wahrgenommener oder anderer Events einfließt
- Alle Messgrößen, die implantatabhängige Signale aufzeichnen
- Alle Messgrößen, die Extrasystolen unabhängig ihres Ursprungorts bestimmen
- Alle Messgrößen, die die Hämodynamik eines Patienten oder sonstige Elastizitäten, Drücke, Volumina oder Abstände erfassen
- Alle Messgrößen, die außerhalb eines Implantats bestimmt werden, wie Größen, die durch aus Funk angebundenen Sensoren gewonnen werden oder Größen von Geräten, die Daten außerhalb des Körpers aufzeichnen und diese telemetrisch der Auswertungseinheit übermitteln
- Alle biomedizinischen Messgrößen wie Stimulationsschwellen, Elektrodenkonfigurationen, Sensorverstärkungen oder Versatzwerte
- Alle Messgrößen wie Blutzuckerspiegel, andere Biomarker oder ähnliche Größen
- Alle Messgrößen, die biometrische Informationen über den Patienten bestimmen
- Alle Messgrößen, die zusätzliche Informationen über eine Medikamentierung aufzeichnen
- Alle Messgrößen die aus elektrophysiologischen oder biochemischen Verfahren bestimmt werden
- Alle Messgrößen, die Signale aus bildgebenden, akustischen oder mechanischen Verfahren erfassen

- Alle oben genannten Messgrößen, die zuvor normiert und/oder skaliert wurden
- Mögliche Kombinationen mehrerer der oben genannten Messgrößen

Dadurch ergibt sich für oben genannten Parameterwerte, z.B. folgende Klassifikationsfunktion:

$$C_{Bsp} = \sum_{j=1}^{3} \left[ \sum_{k=1}^{4} \left( \alpha_{k,j}^{-} T_{k,j}^{-} + \alpha_{k,j}^{+} T_{k,j}^{+} \right) \right],$$

wobei

$$T_{1,j}^{\pm} = \frac{1}{7} \sum_{L=L_j^{min}}^{L_j^{max}} \frac{1}{3} \left( \tilde{T}_{Par1}^{\pm}(L) + \tilde{T}_{Par2}^{\pm}(L) + \tilde{T}_{Par3}^{\pm}(L) \right)$$

$$T_{2,j}^{\pm} = \frac{1}{7} \sum_{L=L_j^{min}}^{L_j^{max}} \tilde{T}_{Par4}^{\pm}(L)$$

$$T_{3,j}^{\pm} = \frac{1}{7} \sum_{L=L_j^{min}}^{L_j^{max}} \frac{1}{3} \left( \tilde{T}_{Par5}^{\pm}(L) + \tilde{T}_{Par6}^{\pm}(L) + \tilde{T}_{Par7}^{\pm}(L) \right)$$

$$T_{4,j}^{\pm} = \frac{1}{7} \sum_{L=L_j^{min}}^{L_j^{max}} \tilde{T}_{Par8}^{\pm}(L)$$

In diesem Beispiel werden die verschiedenen Beiträge der Primärparameter in den einzelnen Gruppen gleich gewichtet, die erste Parametergruppe beinhaltet z.B. drei rhythmologische Größen (hier mit Par1 bis Par3 bezeichnet), die zweite eine Aktivitätsgröße (Par4), die dritte drei intrakardiale Impedanzgrößen (Par5 bis Par7) und die vierte eine intrathorakale Impedanzgröße (Par8). Die Werte der $\alpha_{k,j}^{\pm}$ wurden nach Vorgabe diskreter Werte im Trainingsprozess wie weiter oben beschrieben optimiert. Eine genauere Spezifikation der Primärparameter ist für das Verständnis des Ausführungsbeispiels nicht nötig.

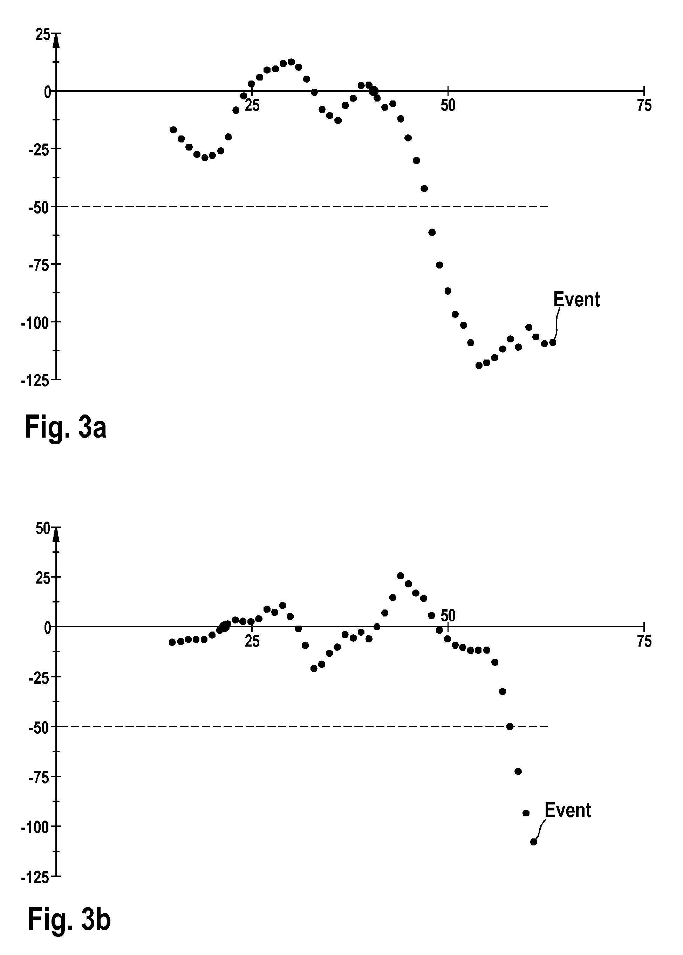

Figuren 3a und 3b zeigen exemplarisch den graphischen Verlauf des bestimmten Prädiktorwertes in willkürlichen Einheiten über die Zeit in Tagen für zwei Patienten. Dabei ist der Zeitpunkt eines follow-ups jeweils mit einem großen Kreis gekennzeichnet, der Schwellenwert mit einer gestrichelten Linie. In Figur 3a wurde das Event 16 Tage vor Eintreten erkannt, in Figur 3b drei Tage zuvor. Mit einem entsprechend geeigneten Alarmsystem wäre in beiden Fällen ein vorheriges Eingreifen durch den Arzt möglich gewesen.

Die Erfindung beschränkt sich in ihrer Ausführungsform nicht auf die vorstehend angegebenen bevorzugten Ausführungsbeispiele. Vielmehr ist eine Anzahl von Varianten denkbar, die von der erfindungsgemäßen Anordnung, dem erfindungsgemäßen Verfahren, dem erfindungsgemäßen Computerprogramm und dem erfindungsgemäßen entsprechenden computerlesbaren Speichermedium auch bei grundsätzlich anders gearteten Ausführungen Gebrauch macht.

**Patentansprüche**

1. Verfahren zur Vorhersage mindestens eines Systemereignisses, wobei
   Messwerte $s_j$ mindestens eines Primärparameters $S_j$ innerhalb mindestens eines Zeitfensters der Länge $L$ erfasst werden,
   für zumindest einen Teil der Zeitfenster jeweils die Häufigkeit $\tau$ eines Trends für zumindest einen Teil des mindestens einen Primärparameters $S_j$ bestimmt wird, und auf Basis der Häufigkeit $\tau$ eines Trends eine Signalisierung eines Systemereignisses erfolgt und/oder systemereignisbezogene Maßnahmen initiiert werden.

2. Verfahren nach Anspruch 1,
   **dadurch gekennzeichnet, dass**
   zumindest ein Teil der Zeitfenster jeweils in mehrere Intervalle unterteilt wird und die Häufigkeit $\tau_i$ des Trends innerhalb der Intervalle für zumindest einen Primärparameter $S_j$ bestimmt wird.

3. Verfahren nach Anspruch 2,
   **dadurch gekennzeichnet, dass**
   die Unterteilung der Fenster in Intervalle in Abhängigkeit eines Primärparameters $S_j$ erfolgt.

4. Verfahren nach einem der Ansprüche 2 oder 3,
   **dadurch gekennzeichnet, dass**
   die Häufigkeiten $\tau$ und/oder $\tau_i$ für eine Anzahl von Fenstern gemittelt werden.

5. Verfahren nach einem der voranstehenden Ansprüche,
   **dadurch gekennzeichnet, dass**
   Gruppen von Primärparametern $S_j$ gebildet werden, und für mindestens eine Gruppe $q$ die Häufigkeiten $\tau$ und/oder $\tau_i$ für eine Anzahl von Fenstern gemittelt werden.

6. Verfahren nach einem der voranstehenden Ansprüche,
   **dadurch gekennzeichnet, dass**
   die Bestimmung des Trends die Prüfung einer Hypothese auf das Vorliegen des Trends umfasst.

7. Verfahren nach einem der voranstehenden Ansprüche,
   **dadurch gekennzeichnet, dass**
   es sich bei dem Trend um einen auf- oder absteigenden Trend handelt.

8. Verfahren nach einem der Ansprüche 4 bis 7,
   **dadurch gekennzeichnet, dass**
   die gemittelten Häufigkeiten $\tau$ und/oder $\tau_i$ in einem Klassifikationsverfahren ausgewertet werden und anhand der Klassifikation die Vorhersage des mindestens einen Systemereignisses erfolgt.

9. Anordnung mit mindestens einem Chip und/oder Prozessor, wobei die Anordnung derart eingerichtet ist, dass ein Verfahren zur Vorhersage mindestens eines Systemereignisses gemäß einem der Ansprüche 1 bis 8 ausführbar ist.

10. Anordnung nach Anspruch 9,
    **dadurch gekennzeichnet, dass**
    die Anordnung Sonden und/oder Sensoren umfasst zur systeminternen und/oder systemexternen Erfassung von Messwerten $s_j$ mindestens eines Primärparameters $S_j$.

11. Anordnung nach Anspruch 9 oder 10,
    **dadurch gekennzeichnet, dass**
    die Anordnung ein aktives oder passives Implantat zur Erfassung der Messwerte $s_j$ umfasst.

**12.** Anordnung nach einem der Ansprüche 9 bis 11,
**dadurch gekennzeichnet, dass**
die Anordnung derart eingerichtet ist, dass zumindest ein Teil der erfassten Messwerte $s_j$ in dem Implantat und/oder einer von dem Implantat entfernt angeordneten Datenverarbeitungseinrichtung ausgewertet werden.

**13.** Computerprogramm, das es einer Datenverarbeitungseinrichtung ermöglicht, nachdem es in Speichermittel der Datenverarbeitungseinrichtung geladen worden ist, ein Verfahren zur Vorhersage mindestens eines Systemereignisses gemäß einem der Ansprüche 1 bis 8 durchzuführen.

**14.** Computerlesbares Speichermedium, auf dem ein Programm gespeichert ist, das es einer Datenverarbeitungseinrichtung ermöglicht, nachdem es in Speichermittel der Datenverarbeitungseinrichtung geladen worden ist, ein Verfahren zur Vorhersage mindestens eines Systemereignisses gemäß einem der Ansprüche 1 bis 8 durchzuführen.

**15.** Verfahren, bei dem ein Computerprogramm nach Anspruch 13 aus einem elektronischen Datennetz, wie beispielsweise aus dem Internet, auf eine an das Datennetz angeschlossene Datenverarbeitungseinrichtung heruntergeladen wird.

L = L_min, ..., L_max

H = H_min, ..., H_max

Event

39   36   33   30   27   24   21   18   15   12   9   6   3   0

Tage vor dem Ereignis

# Fig. 1

Messgröße $S_j = \{y_1, y_2, y_3, ...\}_j$

Fenster der Länge L mit Abstand H vom Fensterende bis zum vorherzusagende Ereignis

Nächstes Fenster

Messsignale der Messgröße $S_j$

Gültig?

nein

ja

Berechnung von $C(L) = \sum\limits_{k=1}^{n-1} \sum\limits_{l=k+1}^{n} sign(y_l - y_k)$

Berechnung der Trendwahrscheinlichkeit p im Fenster mittels Kendall-Statistik

$C(L) \gtrless \pm K_{n,p}$

Schleife über H und L

$\hat{T}^-$ - Prozent der Fenster, die einen Abwärtstrend aufweisen mit der Wahrscheinlichkeit $p \geq p_0$

$\hat{T}^+$ - Prozent der Fenster, die einen Abwärtstrend aufweisen mit der Wahrscheinlichkeit $p \geq p_0$

## Fig. 2

Fig. 3a

Fig. 3b

| Europäisches Patentamt<br>European Patent Office<br>Office européen des brevets | **EUROPÄISCHER RECHERCHENBERICHT** | **Nummer der Anmeldung**<br>EP 10 15 7114 |
| --- | --- | --- |

### EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
| --- | --- | --- | --- |
| L | Die in der Anmeldung (Art 92 EPÜ) definierten technischen Aspekte werden als allgemein bekannt angesehen. Wegen ihrer Bekanntheit wird kein schriftlicher Nachweis für notwendig erachtet. Weitere Einzelheiten finden sich in der beiliegenden Stellungnahme und in Referenz (XP002456252).<br>----- | 1-15 | INV.<br>G06F19/00 |
| | | | **RECHERCHIERTE SACHGEBIETE (IPC)**<br><br>G06F |

Der vorliegende Recherchenbericht wurde für alle Patentansprüche erstellt

| Recherchenort | Abschlußdatum der Recherche | Prüfer |
| --- | --- | --- |
| München | 27. Mai 2010 | Samulowitz, Michael |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

.........................................................................
& : Mitglied der gleichen Patentfamilie, übereinstimmendes Dokument

EPO FORM 1503 03.82 (P04C03)